# EUROPEAN PATENT SPECIFICATION

(11) **EP 3 833 696 B1**
(45) Date of publication and mention of the grant of the patent: **14.09.2022**
(21) Application number: 19758860.1
(22) Date of filing: 05.08.2019
(51) Int. Cl.: C08F 8/00, C12Q 1/689, G01N 15/14, C12Q 1/6869, B01L 3/00, C08F 8/12, C08F 8/32, C08F 8/14, C08F 8/48, C08F 8/34

(54) **FLOW CELLS WITH STABLE POLYMER COATING AND THEIR USES FOR GENE SEQUENCING**
DURCHFLUSSZELLEN MIT STABILER POLYMERBESCHICHTUNG UND IHRE VERWENDUNG ZUR GEN SEQUENZIERUNG
CELLULES A ECOULEMENT AVEC UN REVETEMENT POLYMERE STABLE ET LEURS UTILISATIONS POUR LE SEQUENCAGE DE GENES

(30) Priority: 06.08.2018 US 201862715067 P
(43) Date of publication of application: 16.06.2021
(73) Proprietor: Corning Incorporated, Corning, New York 14831 (US)
(72) Inventor: FANG, Ye, Painted Post, New York 14870 (US); LYNN, Jeffrey Glenn, Wellsboro, Pennsylvania 16901 (US); SUN, Wei, Painted Post, New York 14870 (US); WEI, Ying, Painted Post, New York 14870 (US)
(74) Representative: Elkington and Fife LLP
(86) International application number: PCT/US2019/045045
(87) International publication number: WO 2020/033287

(56) References cited:
- WO-A1-2017/015018
- WO-A1-2019/055924
- US-A1- 2015 005 447
- US-A1- 2015 038 039

## Description

### FIELD OF DISCLOSURE

This disclosure is related to microfluidic flow cell devices and methods of manufacturing and using microfluidic devices for biomolecular analysis, in particular, gene sequencing.

### BACKGROUND

A wide variety of biomolecular analysis techniques, such as DNA microarrays, next generation sequencing (NGS), or DNA based biosensors, employ synthetic DNA probe molecules as the most critical detection element. These DNA probe molecules are often required to be covalently attached to a solid support including flat two-dimensional surfaces and three-dimensional surfaces such as, for example, micro-beads and micro/nano-particles. The covalent attachment of these DNA probe molecules on a surface can be achieved using a variety of different approaches. Regardless of the approach used, these gene sequencing techniques all come with many sequencing cycles, each of which involves multiple rigorous washing steps, which, in turn, often lead to a loss of DNA molecules over time, thus limiting the read length to be achieved.

Optical detection based NGS techniques typically employ a high density DNA primer (also termed as primer lawn) to capture DNA fragments, all having an adaptor sequence that are complementary to the DNA primer sequence, from a biological sample. Once DNA fragments are captured, all DNA fragments are locally amplified to form, ideally, monoclonal clusters. After clustering, the sequence of the DNA fragment within each cluster is determined by synthesis, which consists of hundreds of cycles, each cycle consisting of nucleotide addition using a DNA polymerase, followed by washing, imaging, terminator cleavage, and washing. Thus, DNA sequencing by synthesis encounters vigorous, multi-step treatments within the microfluidic devices (e.g., flow cells) where the attachment of DNA to the reaction substrate can be problematic.

Accordingly, there is a need for improved techniques and corresponding chemistries to more effectively couple DNA fragments to a variety of different substrates used in microfluidic flow cell devices that can withstand the cycling conditions used in synthetically sequencing DNA strands. The development of new chemistries that can provide more stable linkages between DNA fragments and the substrates of flow cell devices can translate into improved reliability, accuracy, and manufacturing costs to produce longer DNA sequences.

### SUMMARY

According to some aspects of the present disclosure, a flow cell article includes: a substrate having one or more layers; a fluidic channel disposed in the substrate wherein the fluidic channel includes at least one reactive surface having: a coupling agent with a first functional group covalently attached to the substrate of the fluidic channel and a second imide functional group covalently attached to a polymer of formula (I): 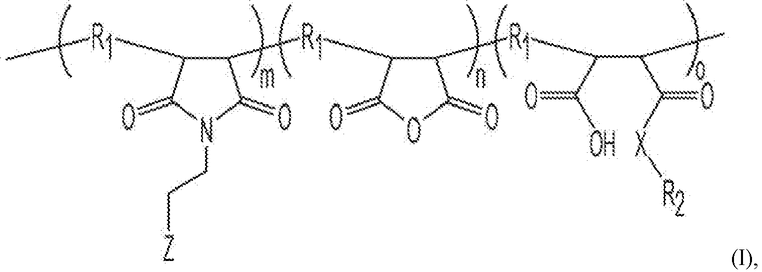 where: R₁ is a residue of an unsaturated monomer that has been copolymerized with maleic anhydride; R₂ is H, an alkyl group, an oligo(ethylene glycol), and/or a dialkyl amine; m, n, and o are each from 1 to 10,000; X is a divalent NH, O, and/or S; and Z is the first functional group.

According to some aspects of the present disclosure, a flow cell system includes: a substrate having one or more layers; a fluidic channel disposed in the substrate wherein the fluidic channel includes at least one reactive surface having: a coupling agent with a first functional group covalently attached to the substrate of the fluidic channel and a second functional group positioned away from the substrate; a polymer of formula (II) to be covalently attached to the second functional group of the coupling agent: 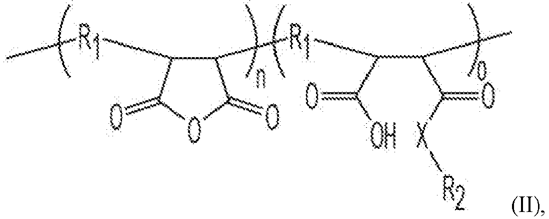 where: R₁ is a residue of an unsaturated monomer that has been copolymerized with maleic anhydride; R₂ is H, an alkyl group, an oligo(ethylene glycol), and/or a dialkyl amine; n and o are each an integer from 1 to 10,000; and X is a divalent NH, O, and/or S; a nucleic acid primer molecule covalently attached to the polymer.

According to other aspects of the present disclosure, a method of making a flow cell article is provided. The method includes the steps of: contacting a fluidic channel disposed in a substrate with a coupling agent to covalently attach a first functional group to the fluidic channel; contacting a polymer of formula (II) with the coupling agent to covalently attach the polymer to a second functional group to form an imide linkage on a tethered polymer; 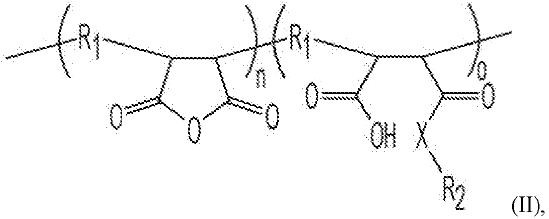 where: R₁ is a residue of an unsaturated monomer that has been copolymerized with maleic anhydride; R₂ is H, an alkyl group, an oligo(ethylene glycol), and/or a dialkyl amine; n and o are each an integer from 1 to 10,000; and X is a divalent NH, O, and/or S; contacting a nucleic acid primer molecule with the tethered polymer to covalently attached to the nucleic acid primer molecule to the tethered polymer.

According to still other aspects of the present disclosure, a method for sequencing nucleic acids is provided. The method includes the steps of: providing a flow cell article having: a substrate including one or more layers; a fluidic channel disposed in the substrate wherein the fluidic channel includes at least one reactive surface comprising a coupling agent having a first functional group covalently attached to the substrate of the fluidic channel and a second imide functional group covalently attached to a polymer of formula (I): 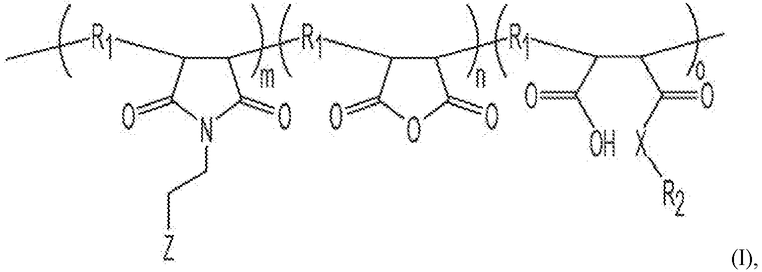 where: R₁ is a residue of an unsaturated monomer that has been copolymerized with maleic anhydride; R₂ is H, an alkyl group, an oligo(ethylene glycol), and/or a dialkyl amine; m, n, and o are each from 1 to 10,000; X is a divalent NH, O, and/or S; and Z is the second functional group; contacting a nucleic acid primer molecule with the polymer of formula (I) to covalently attached the nucleic acid primer molecule to the polymer of formula (I); capturing DNA fragments using the nucleic acid primer molecule, wherein each DNA fragment contains a complementary sequence to the nucleic acid primer molecule; and adding nucleotides to the end of the nucleic acid primer molecule to synthesize a complementary DNA sequence for each DNA fragment captured wherein the complementary DNA sequence is covalently coupled to the polymer of formula (I) through the nucleic acid primer molecule.

Additional features and advantages will be set forth in the detailed description which follows, and in part will be readily apparent to those skilled in the art from that description or recognized by practicing the embodiments as described herein, including the detailed description which follows, the claims, as well as the appended drawings.

It is to be understood that both the foregoing general description and the following detailed description are merely exemplary, and are intended to provide an overview or framework to understanding the nature and character of the claims. The accompanying drawings are included to provide a further understanding, and are incorporated in and constitute a part of this specification. The drawings illustrate one or more embodiments, and together with the description serve to explain principles and operation of the various embodiments.

### BRIEF DESCRIPTION OF THE DRAWINGS

The following is a description of the figures in the accompanying drawings. The figures are not necessarily to scale, and certain features and certain views of the figures may be shown exaggerated in scale or in schematic in the interest of clarity and conciseness.
FIG. 1 is a schematic view of a flow cell article covalently coupled to DNA probe molecules according to some aspects of the presentdisclosure;
FIG. 2 is a schematic view of a polymer coating covalently bonded to a coupling agent attached to a substrate forming a fluidic channel according to some aspects of the present disclosure;
FIG. 3 is a schematic view of a polymer coating covalently bonded to a coupling agent attached to a metal oxide layer coated on a substrate forming a fluidic channel according to some aspects of the present disclosure;
FIGS. 4A-4C are scanning electron microscopic images of a fluidic channel surface having a polymer coating according to some aspects of the present disclosure;
FIGS. 5A and 5B respectively show a photo image (5A) and fluorescent (5B) image of a flow cell article having eight channels, each consisting of an amine-terminated dA30 attached to a polymer coating, after hybridizing a Cy3 dye labeled dT30, according to some aspects of the present disclosure; and
FIG. 6 is a graph showing the comparative difference in sequencing read lengths for an amide linkage and an imide linkage used to covalently bong a coupling agent to a polymer coating used to attach DNA.

### DETAILED DESCRIPTION

Additional features and advantages will be set forth in the detailed description which follows and will be apparent to those skilled in the art from the description, or recognized by practicing the embodiments as described in the following description, together with the claims and appended drawings.

As used herein, the term "and/or," when used in a list of two or more items, means that any one of the listed items can be employed by itself, or any combination of two or more of the listed items can be employed. For example, if a composition is described as containing components A, B, and/or C, the composition can contain A alone; B alone; C alone; A and B in combination; A and C in combination; B and C in combination; or A, B, and C in combination.

In this document, relational terms, such as first and second, top and bottom, and the like, are used solely to distinguish one entity or action from another entity or action, without necessarily requiring or implying any actual such relationship or order between such entities or actions.

Modifications of the disclosure will occur to those skilled in the art and to those who make or use the disclosure. Therefore, it is understood that the embodiments shown in the drawings and described above are merely for illustrative purposes and not intended to limit the scope of the disclosure, which is defined by the following claims, as interpreted according to the principles of patent law, including the doctrine of equivalents.

For purposes of this disclosure, the term "coupled" (in all of its forms: couple, coupling, coupled, etc.) generally means the joining of two components directly or indirectly to one another. Such joining may be stationary in nature or movable in nature. Such joining may be achieved with the two components and any additional intermediate members being integrally formed as a single unitary body with one another or with the two components. Such joining may be permanent in nature, or may be removable or releasable in nature, unless otherwise stated.

As used herein, the term "about" means that amounts, sizes, formulations, parameters, and other quantities and characteristics are not and need not be exact, but may be approximate and/or larger or smaller, as desired, reflecting tolerances, conversion factors, rounding off, measurement error and the like, and other factors known to those of skill in the art. When the term "about" is used in describing a value or an end-point of a range, the disclosure should be understood to include the specific value or end-point referred to. Whether or not a numerical value or end-point of a range in the specification recites "about," the numerical value or end-point of a range is intended to include two embodiments: one modified by "about," and one not modified by "about." It will be further understood that the end-points of each of the ranges are significant both in relation to the other end-point, and independently of the other end-point.

The terms "substantial," "substantially," and variations thereof as used herein are intended to note that a described feature is equal or approximately equal to a value or description. For example, a "substantially planar" surface is intended to denote a surface that is planar or approximately planar. Moreover, "substantially" is intended to denote that two values are equal or approximately equal. In some embodiments, "substantially" may denote values within about 10% of each other, such as within about 5% of each other, or within about 2% of each other.

Directional terms as used herein-for example up, down, right, left, front, back, top, bottom-are made only with reference to the figures as drawn and are not intended to imply absolute orientation.

As used herein the terms "the," "a," or "an," mean "at least one," and should not be limited to "only one" unless explicitly indicated to the contrary. Thus, for example, reference to "a component" includes embodiments having two or more such components unless the context clearly indicates otherwise.

The term "Next Generation Sequencing" or "NGS", as used herein, is defined to include a type of DNA sequencing technology that uses parallel sequencing of many small fragments of DNA from a biological sample to determine a gene sequence. NGS can be used to sequence every nucleotide in a genome, or small portions of the genome such as the exome or a preselected subset of genes.

The past decade has seen extraordinary progress in cataloging human genetic variations, and correlating these variations with susceptibility to disease, responsiveness to specific therapies, susceptibility to dangerous drug side effects, and other medically actionable characteristics. Advances in NGS have led to a decreased cost per megabase and an increase in the number and diversity of genomes sequenced. Key to advances in genome wide sequencing is to use a flow cell to partition millions of DNA fragments generated from a biological DNA sample onto the surface of the flow cell so that almost all fragments that are immobilized can be sequenced simultaneously. Some of NGS technologies, in particular the short-read sequencing techniques, include the covalent immobilization of DNA fragments onto the surface of a flow cell for sequencing. Significant to sequencing efficiency and quality is the ability to achieve stable, reproducible, and optimal attachment of DNA molecules onto the surface of a flow cell.

The embodiments of the present disclosure generally relate to nucleic acid analysis, and more specifically to methods of making and using microfluidic flow cell devices for use in, for example, massively parallel genomics analysis (e.g., next generation sequencing, NGS). Many important molecular applications, such as DNA microarrays, NGS, or DNA based biosensors may use synthetic DNA probe molecules attached to solid supports including flat two-dimensional surfaces, such as glass, silica, or silicon slides, and to three-dimensional surfaces such as micro-beads and micro/nano-particles. The immobilization of DNA probe molecules on a microfluidic surface can typically be achieved using numerous methods, for instance, electrostatic interaction, covalent coupling, entrapment, and like. One technique used to covalently attach a DNA probe to a surface involves the use of a bifunctional linker molecule to link a DNA probe molecule with an amine-presenting surface using amide bonds. For instance, common to many optical detection based gene sequencing methods is the use of a surface containing high density of an oligonucleotide primer or probe, wherein the DNA primer is covalently attached to the surface. However, amide bond based covalent attachment generally comes with relatively low stability with respect to vigorous sequencing cycles, thus only enabling shortread lengths.

The present disclosure provides materials and methods for covalently coupling amine-terminated DNA primer molecules onto a solid support for nucleic acid analysis, in particular gene sequencing. The present disclosure additionally provides a reactive surface to covalently capture amine terminated DNA probe fragments. The fragment density and position can be precisely controlled, so that the synthesis and growth of polyclonal clusters can be spatially controlled and sequenced efficiently with improved quality and precision.

In various aspects of the present disclosure, a flow cell article 10 is provided. The flow cell article 10 includes a substrate 14 having one or more layers 16; a fluidic channel 18 disposed in the substrate 14 where the fluidic channel 18 includes at least one reactive surface 22 having a coupling agent 26 with a first functional group 30 covalently attached to the substrate 14 of the fluidic channel 18 and a second functional group 34 (e.g., primary amine) covalently attached to a polymer 38 using an imide bond, the covalently attached polymer 38 can be represented in formula (I): 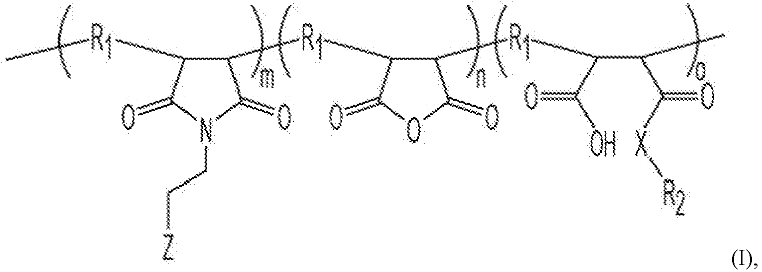 The R₁ is a residue of an unsaturated monomer that has been copolymerized with maleic anhydride. R₂ is H, an alkyl group, an oligo(ethylene glycol), and/or a dialkyl amine. m, n, and o are each from 1 to 10,000. X is a divalent NH, O, and/or S. Z is the first functional group 30.

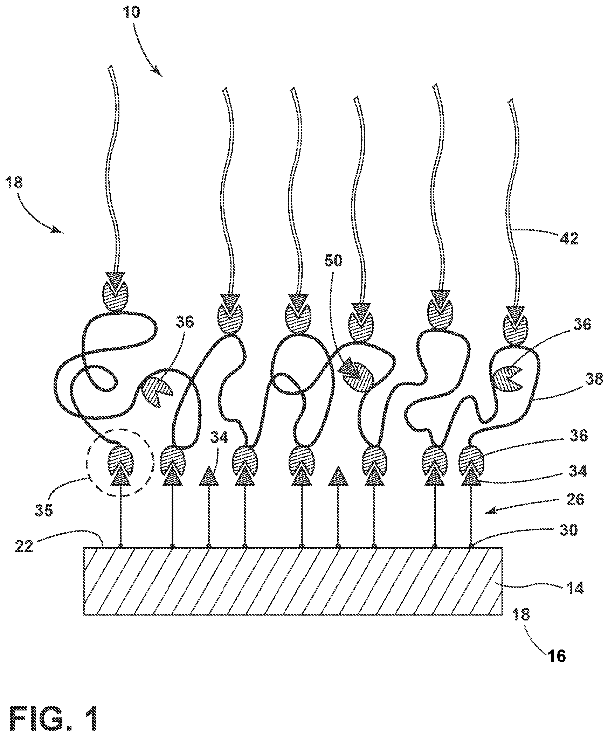

Referring now to FIG. 1, a schematic view of the flow cell article 10 covalently coupled to a plurality of nucleic acid primer molecules 42 according to some aspects of the present disclosure is provided. The solid support or substrate 14 is operatively coupled to the coupling agent 26 where the coupling agent 26 includes the first functional group 30 covalently attached to the substrate 14 and the second functional group 34 covalently attached to the polymer 38. In some aspects, the first functional group 30 of the coupling agent 26 includes an amino silane and/or an amino organophosphate. The polymer 38 includes a connector group 36 that may be used to covalently couple the second functional group 34 to form a polymer linkage 35. In some aspects, the same type of connector group 36 may be used to covalently couple the plurality of nucleic acid primer molecules 42 and second functional group 34 using the same or a different type of polymer linkage 35. As an alternative to the traditionally used more reactive amide or ester linkages, in some aspects, the polymer linkage 35 used in the embodiments disclosed herein may include an imide functional group that can be formed through the condensation reaction between a primary amine and an anhydride function group. In some aspects, the connector group 36 is a succinic anhydride group, the second functional group 34 is a primary amine, and the corresponding polymer linkage group 35 is an imide. The fully synthesized flow cell article 10 includes the substrate 14 covalently bonded to the polymer 38 using the coupling agent 26 in addition to at least one nucleic acid primer molecule 42 covalently coupled to the substrate 14 through the polymer 38. The DNA-bound flow cell article 10 would be positioned to face or extend into the fluidic channel 18 to provide the reactive surface 22 in order to capture or bind desired DNA fragments.

In some aspects, the polymer 38 may include a polymer containing an anhydride functionality. In some aspects, the polymer 38 may include poly(ethylene-co-maleic anhydride) (EMA), polyethylene-graft-maleic anhydride, polypropylene-graft-maleic anhydride, poly(ethylene/maleic anhydride), poly(ethylene-co-ethyl acrylate-co-maleic anhydride, poly(isobutylene-co-maleic anhydride), poly(maleic anhydride-co-1-octadecene), poly(styrene-co-maleic anhydride), poly(styrene-co-maleic acid), or combinations thereof. Each of the copolymers listed above may include alternating copolymers (e.g., ABABAB), block copolymers (e.g., AAABBBAAABBB), and/or random copolymers (e.g., ABBABAABAB) structures. For example, in some aspects, the polymer 38 may include poly(ethylene-co-maleic anhydride) where the ethylene and maleic hydride monomers are bonded together in an alternating, random, or block structure. In some aspects, a relative ratio of m to n (m:n) is from about 0.5 to about 10.

In some aspects, the polymer 38 used to covalently bond and couple the coupling agent 26 is represented below in formula (II): 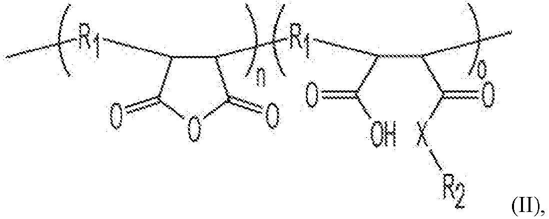 where: R₁ is a residue of an unsaturated monomer that has been copolymerized with maleic anhydride; R₂ is H, an alkyl group, an oligo(ethylene glycol), and/or a dialkyl amine; n and o are each an integer from 1 to 10,000; and X is a divalent NH, O, and/or S. The residue R₁ of the unsaturated monomer would be, for example, a diradical ethyl group when ethylene is copolymerized with maleic anhydride. Once the polymer 38 having formula (II) is reacted with the second functional group 34, the modified structure of polymer 38 is represented below by formula (I): 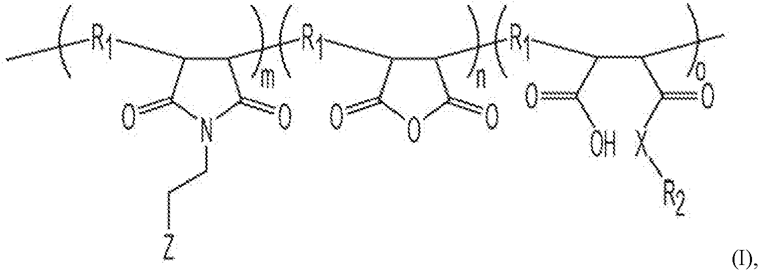 where: R₁, R₂, and X are all as listed above in formula (II); m, n, and o are each an integer from 1 to 10,000; and Z is the first functional group 30. In some aspects, such as with a flow cell system, the nucleic acid primer molecule 42 may be covalently bonded, coupled, and attached to the polymer 38 having formula (I) using the reactive succinic anhydride functionality incorporated into the polymer backbone to form imide linkages with amine groups of the nucleic acid primer molecule 42.

In some aspects, the present disclosure may also provide a flow cell having a DNA probe molecule modified surface, where the density of the DNA probe molecules can be precisely controlled so that excellent DNA hybridization, subsequent polyclonal cluster formation, and sequencing can be achieved. The DNA probe molecule can be covalently attached to the amine reactive polymer coated flow cell surface by incubating the polymer modified flow cell surface with an amine terminated DNA probe molecule in the presence of a modulating second small molecule (e.g., a surface modifying molecule 50) at a specific concentration.

Still referring to FIG. 1, the surface modifying molecule 50 may be used to control the degree (e.g. amount or extent) of the coupling reaction of the coupling agent 26 and/or nucleic acid primer molecule 42 to the polymer 38, which can control or determine the density of the nucleic acid primer molecules attached. The ratio of nucleic acid primer molecules 42 to surface modifying molecules 50 can determine the amount or density of nucleic acid primer molecules 42 positioned at the reactive surface 22. In some aspects, the nucleic acid primer molecule 42 to surface modifying molecule 50 mole ratio (P:S) can be, for example, 0.01:1, 0.1:1, 1:1, 1:2, 1:5, 1:10; 1:20, 1:50, 1:100, 1:1000, 1:10,000, and like ratios, including intermediate values and ranges, depending on the density desired for a given application. For example, in some aspects where clustering can be achieved using the bridge amplification approach, the density of the nucleic acid primer molecule 42 can be relatively low (e.g., 10,000 or 100,000 per square micrometer of surface area in the fluidic channel 18). When clustering is achieved using the template walking approach, the density of nucleic acid primer molecules 42 may be relatively high (e.g., 250,000 or 500,000 per square micrometer of surface area in the fluidic channel 18).

In some aspects, the surface modifying molecule 50 may be an amine containing small molecule. In some aspects, R₂ may be the surface modifying molecule 50. The surface modifying molecule 50 can include, for example, ethanolamine, N,N-dimethylethylenediamine, N,N-diethylethylenediamine, (2-aminoethyl)-trimethylammonium, an amine-terminated polyglycol, and/or oligo-ethylene glycol. The surface modifying molecule 50 may also prevent non-specific binding of biomolecules to the surface of the polymer 38 during sequencing and reduce the background signal over the sequencing cycle. In other aspects, the surface modifying molecule 50 can be introduced during polymer coating or can be introduced during the nucleic acid primer molecule 42 functionalization step. In some aspects, the surface modifying molecule 50 can include, for example, but not necessarily limited to mono-, di-, and tri-aminosilane, such as *y*-aminopropylsilane, 3-(aminopropyl)triethoxysilane (APTES), 3- aminopropyl)trimethoxysilane, 3-aminopropyldimethylmethoxysilane, 3- aminopropyl(diethoxy)methylsilane, aminopropylsilsesquioxane (APS), N-[3- (trimethoxysilyl)propyl]ethylenediamine, N1-(3 trimethoxysilylpropyl)diethylenetriamine, ethylenediamine, propylamine, allylamine, ethanolamine, (2-aminoethyl)trimethylammonium, or combinations thereof.

In some aspects, the nucleic acid primer molecule 42 can be, for example, an amine-terminated nucleic acid or nucleic acid fragment. In some aspects, the nucleic acid primer molecule 42 can have a surface density, for example, of from 1 to 10,000 nucleic acid primer molecules 42 per chain of polymer 38. In other aspects, the nucleic acid primer molecule 42 may have a density of, for example, from 1 to 500,000 nucleic acid primer molecules 42 per square micrometer of surface area in the fluidic channel 18. In still other aspects, the nucleic acid primer molecule 42 may have a density of, for example, from 1,000 to 500,000 nucleic acid primer molecules 42 per square micrometer (*µ*m²) surface area in the fluidic channel 18 when polyclonal clustering is required for sequencing. Alternatively, the nucleic acid primer molecule 42 may have a density, for example, of from 1 to about 1000 nucleic acid primer molecules 42 per *µ*m² surface area in the fluidic channel 18 when single molecule analysis is required for sequencing. In some aspects, the nucleic acid primer molecule 42 can be, for example, 5' -amine terminated dA30, 3' -amine terminated dA30, amine terminated dT30, and like probe molecules. In other aspects, the nucleic acid primer molecule 42 can be substituted with an RNA probe molecule for sequencing RNA.

In some aspects, the flow cell article 10 can include, for example, at least two solid substrates 14 bound together using known techniques in the art including, for example, a laser-assisted process, a tape, a polyimide adhesive, a pressure sensitive adhesive tape, or a combination thereof. In some aspects, the two substrates 14 can be made of the same material or in other aspects, the two substrates can be made of different materials. Depending on the application, the substrate 14 can be, for example, plastic, glass, silicon, fused silica, or quartz. The flow cell article 10 defines a chamber, cavity, and/or fluidic channel 18. The flow cell article 10 can include, for example, ports for media flow directing liquid into and out of the chamber (see Illumina.com; Illumina Sequencing Technology, Spotlight: Illumina^{®} Sequencing). In some aspects, the substrate 14 may include a glass, a glass ceramic, a silicon, a fused silica, a quartz, a thermoplastic, or a thermoset plastic.

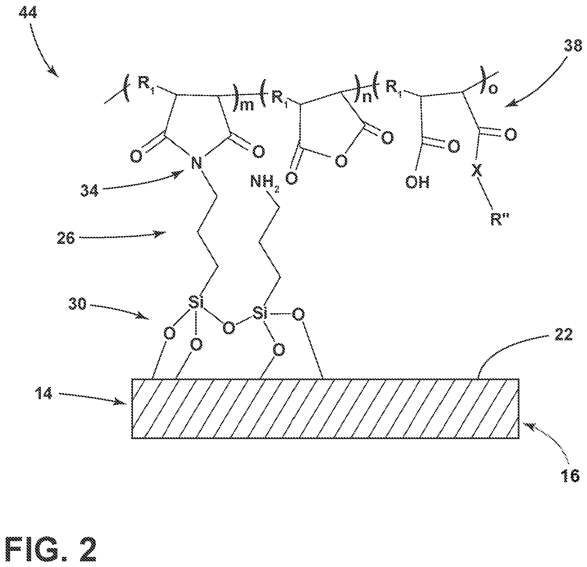

Referring now to FIG. 2, a schematic view of an intermediate microfluidic device 44 including the polymer 38 covalently bonded to the coupling agent 26 attached to the substrate 14 according to some aspects of the present disclosure is provided. The intermediate microfluidic device 46 illustrated in FIG. 2 includes the polymer 38 coated on the substrate surface that would be positioned within the fluidic channel 18. The surface of the substrate 14 positioned in the fluidic channel 18 of the intermediate microfluidic device 44 may be first coated with the coupling agent 26. As illustrated, in some aspects, the coupling agent 26 may include an amino silane. The use of an amino silane as the coupling agent 26 provides a silane to be used as the first functional group 30 and an amine to be used as the second function group 34. Once the first functional group 30 silane is covalently coupled (e.g., forming covalent bonds) to the surface of the substrate 14, the amine used as the second functional group 34 may be covalently coupled to the polymer 38. In some aspects, the connector group 36 (see FIG. 1) or succinic anhydride moiety on the polymer 38 used to couple the amine may be condensed to form an imide linkage or imide bond with a water molecule evolved in the condensation reaction. The resulting polymer 38 covalently coupled to the coupling agent 26 to form a coating on the substrate 14 contains three different types of functional groups: 1) imide groups covalently coupling the coupling agent 26 to the polymer 38; 2) amine-reactive anhydride groups (e.g., succinic anhydride groups) incorporated into the polymer backbone that support coupling agent 26 and DNA fragment 42 attachment; and 3) modifier functional groups (e.g., ester, amine, carboxylic acid, carboxylate, and/or thioesters) used to facilitate tuning the DNA density and surface properties.

Still referring to FIG. 2, the coupling agent 26 may include an amino silane, while the solid substrate can be glass, glass ceramics, silicon, fused silica, thermoplastic, thermoset, and/or quartz. The amino silane may include a molecule including both primary amine and silane functional groups, typically connected to each other using for example, an alkyl, alkoxy, and/or glycol group. In some aspects, the amino silane may include 3-aminopropyltrimethoxylsilane, 3-aminopropyltriethoxysilane, 3-(2-aminoethyl)-aminopropyltrimethoxysilane, aminopropylmethyldialkoxysilanes, 4-aminobutyltriethoxysilane, 4-amino-3,3-dimethylbutyltrimethoxysilane, N-(6-aminohexyl)aminomethyltriethoxysilane, or combinations thereof. In some aspects, the silane of the amino silane can include, for example, a silsesquioxane, or a mixture thereof. In other aspects, the amino silane can include, for example, 3-(aminopropyl)triethoxysilane, and the silsesquioxane can be, for example,aminopropylsilsesquioxane.

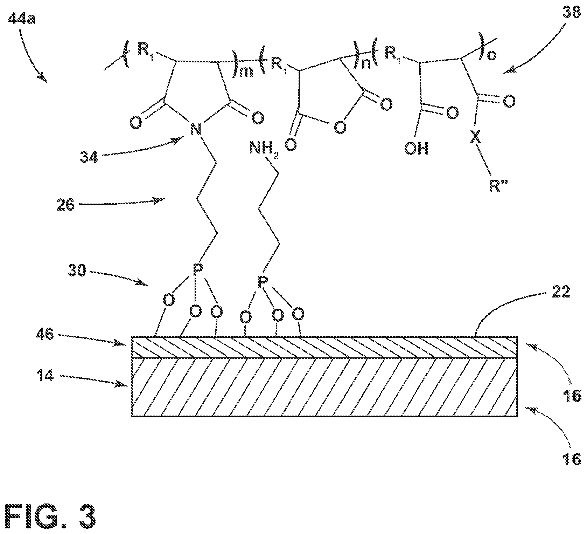

Referring now to FIG. 3, a schematic view of an intermediate microfluidic device 44a including the polymer 38 covalently bonded to the coupling agent 26 attached to a metal oxide layer 46 positioned on the substrate 14 according to some aspects of the present disclosure is provided. The intermediate microfluidic device 44a includes the polymer 38 coated on the substrate surface. In some aspects, the surface of the substrate 14 may be first coated with the metal oxide layer 46 of the intermediate microfluidic device 44a where the metal oxide layer 46 may then be coated with the coupling agent 26. As illustrated, in some aspects, the coupling agent 26 may include an amino organophosphate. The use of an amino organophosphate as the coupling agent 26 provides an organophosphate to be used as the first functional group 30 and an amine to be used as the second functional group 34. Once the first functional group 30 or organophosphate is covalently coupled (e.g., forming covalent bonds) to the metal oxide layer 46 surface of the substrate 14, the second functional group 34 or amine may be covalently coupled to the polymer 38. In some aspects, the connector group 36 (see FIG. 1) or succinic anhydride moiety on the polymer 38 used to couple the amine may be condensed to form an imide linkage or imide bond with a water molecule evolved in the condensation reaction.

Still referring to FIG.3, the coupling agent 26 may include an amino organophosphate where the solid substrate 14 is coupled or coated with the metal oxide layer 46. The metal oxide used in the metal oxide layer 46 may include, for example, but is not limited to, Al₂O₃, ZnO₂, Ta₂O₅, Nb₂O₅, SnO₂, MgO, indium tin oxide, CeO₂, CoO, Co₃O₄, Cr₂O₃, Fe₂O₃, Fe₃O₄, In₂O₃, Mn₂O₃, NiO, a-TiO₂ (anatase), r-TiO₂ (rutile), WO₃, Y₂O₃, ZrO₂. In some aspects, the metal oxide layer 46 is transparent within a visible wavelength range (e.g., from 400 to 750nm). For example, the metal oxide layer 46 can have a transmission of at least 50%, 60%, 70%, 80%, 90%, 95%, or 99% within the visible wavelength range.

In some aspects, the amino organophosphate may include a molecule having at least one or both a primary amine and organophosphate functional group. The amine and organophosphate functionalities may be coupled to each other using, for example, an alkyl, alkoxy, and/or glycol group. The amino organophosphate may include 3-aminopropyl dihydrogen phosphate, 4-aminophenyl phosphate, 2-aminoethyl dihydrogen phosphate, 2-(3- aminopropyl)aminoethyl phosphorothioate, or a combination thereof.

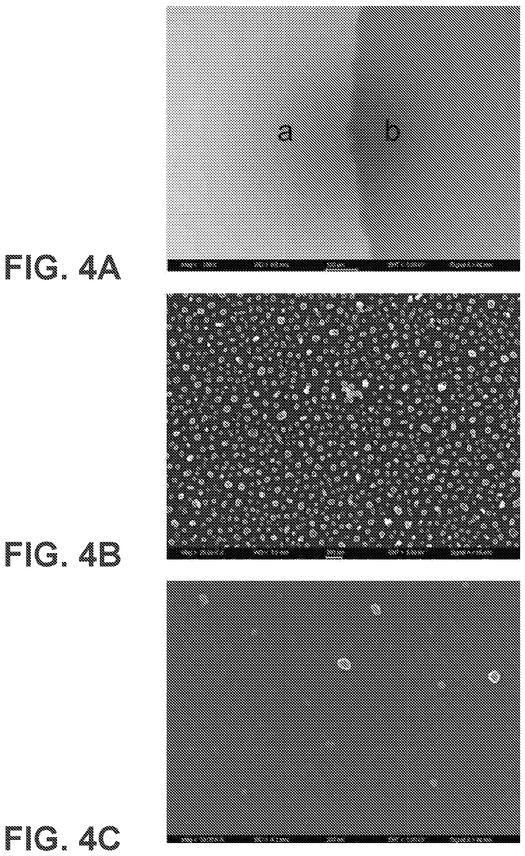

Referring now to FIGS. 4A-4C, scanning electron microscopic images of a fluidic channel surface having a polymer coating according to some aspects of the present disclosure are provided. The three presented scanning electron microscopic images of the surface of the micro fluidic channel 18 having a stable polymer coating that is partially treated with an amine terminated dA30 primer DNA include: (A) a low resolution SEM image of the surface showing two regions: left (a) and right (b); (B) a high resolution SEM image of the left part of the surface A showing that the polymer is present as a nanoparticle; and (C) a high resolution SEM image of the right part of the surface A after reacted with the dA30, showing that the majority of the polymer nanoparticles are open to form a homogeneous dA30-polymer coating on the surface.

Still referring to FIGS. 4A-4C, the polymer 38, once covalently attached to the surface using primarily imide bonds, can be in nanoparticle format, in particular single-chain nanoparticle format, instead of extended polymer chain format. The nanoparticle coating scheme is enabled by incubating the amine-presenting surface with the polymer in a mixture of organic solvents, wherein each single polymer is self-assembled into a nanoparticle. The mixture of organic solvents comprises consists essentially of, or consists of a polymer-soluble solvent such as N-methyl-2- pyrrolidone (NMP) and a polymer-insoluble solvent such as isopropyl alcohol (IPA). As shown in FIGS. 4A and B, the polymer covalently attached to the surface primarily using imide bond with the coupling agent comprises, consists essentially of, or consists of individual nanoparticles. However, once functionalized with the DNA primer (here, 5'-amino-C6-dA30), the polymer nanoparticles become open and cover the entire surface to form a homogeneous coating as shown in FIGS. 4A and 4C. Because DNA is negatively charged, self-repulsion among DNA molecules will cause expansion of polymer molecules to cover an entire surface, even though there are spaces between polymer nanoparticles attached. Also noticeable is well-distribution of polymer nanoparticles after being attached to the substrate. Without being bound by any theory, this is believed to be due to well-known crowding effect of polymer particles in solvent. Sequencing by synthesis using the dA30 functionalized surface, together with template walking based clustering and reversible terminator DNA synthesis chemistry, showed that the EMA nanoparticle coated surface enables 75 reads in average, while the extended EMA polymer chain coated surface only permitted a few reads (<10 bp) in average. By controlling the concentration of polymer 38 and reaction duration used for coating, the density of polymer nanoparticles covalently attached to the substrate can be controlled. Lower concentrations of polymer 38 used for the coating process can result in lower density of polymer nanoparticles attached. Based on the polymer crowding effect and the limited surface coverage of each polymer after expansion induced by the coupled DNA primer molecules, the polymer coated substrate can be viewed as a patterned surface, even though the polymer nanoparticles can have different size (as shown in FIG. 4B). Thus, when the density of polymer nanoparticles attached is low (e.g., 1 polymer nanoparticle per 10,000 nm², or per 20,000 nm², or per 40,000 nm², or per 100,000 nm², or per 250,000 nm², or per 1000,000 nm²), one can generate a single cluster within an area covered by a single polymer nanoparticle, thus effectively forming a random, but well-separated, cluster array. This is in particular useful for high density patterned sequencing applications.

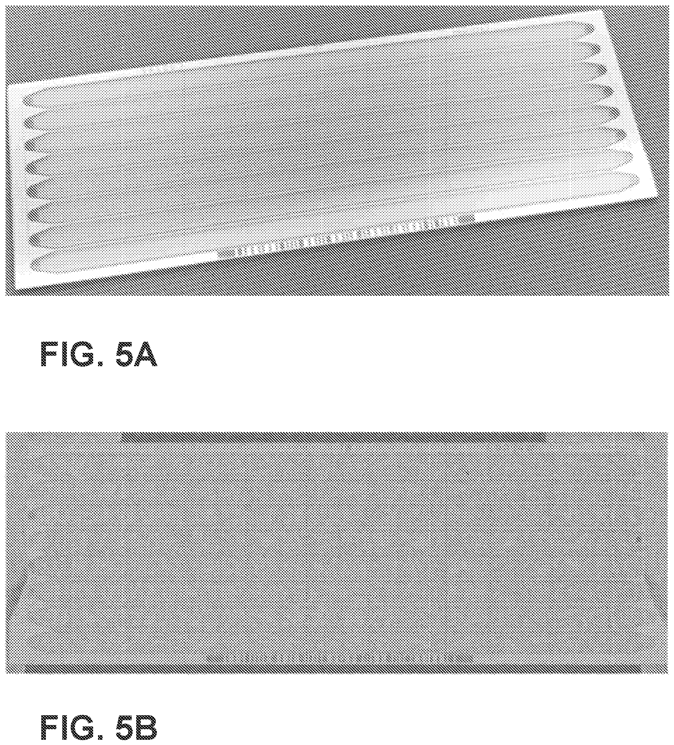

Referring now to FIGS. 5A-5B, a photo image (5A) and fluorescent image (5B) of a flow cell article having eight channels, each comprising, consisting essentially of, or consisting of an amine-terminated dA30 attached to a polymer coating according to some aspects of the present disclosure is provided. FIG. 5A shows a photo image of an entire flow cell, and FIG. 5B shows a confocal fluorescent image of an entire flow cell having 8 channels, each comprising, consisting essentially of, or consisting of amine-terminated dA30 attached to a reactive polymer coating, after being hybridized with Cy3-labeled dT30.
Specifically, all channels are first coated withy (gamma)-aminopropylsilane, followed by covalent attachment of propylamine-derivatized poly(ethylene-alt-maleic anhydride). Afterwards, all channels were incubated with 50 microM 5'-amineterminated dA30 in the presence of 100 microM ethanolamine, followed by further treatment as mentioned below. Channel 1 to 8 from top to bottom are: Channel 1, 2, 7, and 8: rinsed with phosphate buffer (PBS) three times; Channel 3, and 6: incubated with 0.05 M NaOH 5 min, rinsed with PBS three times; and Channel 4, and 5: rinsed with 60°C water five times, 1 min each. Finally, all channels were incubated with 1 microM Cy3-labeled dT30 for 45 min. After rinsing with PBS three times and dried, confocal microscopy was used to scan the entire flow cell. All fluorescence images collected were assembled together to form the fluorescent image of the entire flow cell as shown.

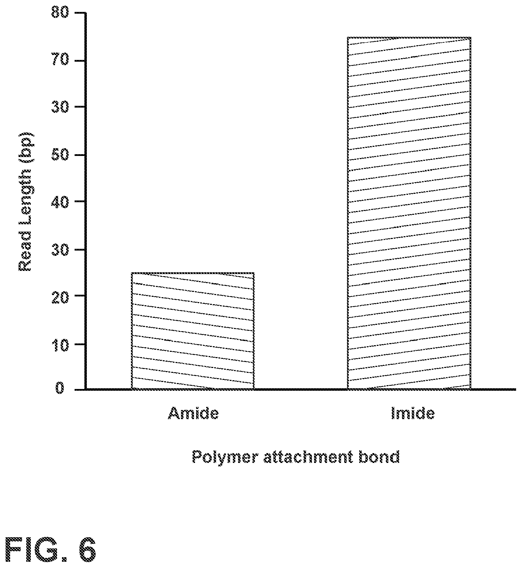

Referring now to FIG. 6, a graph showing the comparative difference in sequencing read lengths for an amide linkage and an imide linkage used for covalently bonding a coupling agent to a polymer coating used to attach DNA is provided. The graph illustrates the comparative differences in sequencing read length for two polymer coatings: one polymer attached using an amide bond linkage; and another polymer attached using an imide bond linkage.

In some aspects of the present disclosure, the imide bonds between the anhydride containing polymer 38 and the amine terminal group of the coupling agent 26 is formed using a two-step sequential reaction scheme. First, an anhydride group of the polymer reacts with an amine group on the surface to form an amide bond, followed by ring closure to form an imide bond via heat treatment (120 to 150 °C for several hours). As shown in FIG. 6, sequencing by synthesis using the dA30 functionalized surface, together with template walking based clustering and reversible terminator DNA synthesis chemistry, showed that the polymer covalently attached to the amine presenting surface via *imide* bonds permitted 75 reads in average, while the polymer covalently attached to the amine presenting surface via *amide* bonds only enabled 25 reads in average. Further optimization of DNA attachment (e.g., density and distribution) can further greatly increase read length (e.g., up to 200 reads for single end sequencing), considering the high stability of imide bond against harsh treatment (e.g., NaOH, vigorous washing, temperature cycling between room temperature to high temperature). Here, the microfluidic channel surfaces were treated with the same coating and functionalized protocols, except that a 3-hour 120°C heat treatment was used to drive the imide bond formation.

In some aspects of the present disclosure, a method of making the flow cell article 10 is provided. The method includes: contacting the fluidic channel 18 disposed in the substrate 14 with the coupling agent 26 to covalently attach the first functional group to the fluidic channel 18; contacting the polymer 38 of formula (II) with the coupling agent 26 to covalently attach the polymer 38 to the second functional group 34 to form an imide linkage on a tethered polymer; 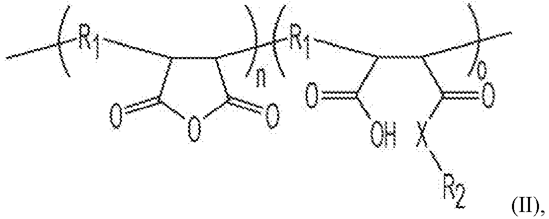 where: the R₁ is a residue of an unsaturated monomer that has been copolymerized with maleic anhydride; R₂ is H, an alkyl group, an oligo(ethylene glycol), and/or a dialkyl amine; n and o are each an integer from 1 to 10,000; and X is a divalent NH, O, and/or S. The method may further include contacting the nucleic acid primer molecule 42 with the tethered polymer 38 (see formula (II)) to covalently attach the nucleic acid primer molecule 42 to the tethered polymer 38. In some aspects, contacting the nucleic acid primer molecule 42 with the tethered polymer 38 may optionally include the presence of the surface modifying molecule 50 to covalently attach the nucleic acid primer molecule 42 to the polymer 38 coupled to the substrate 14 using the coupling agent 26 to form the flow cell article 10. In some aspects, controlling the density of the nucleic acid primer molecules 42 attached to the polymer 38 can be accomplished by using various ratios of the surface modifying molecule 50 with respect to the nucleic acid primer molecule 42.

It is understood that the descriptions outlining and teaching the flow cell article 10 previously discussed, which can be used in any combination, apply equally well to the methods of making and methods of using the flow cell article 10.

In some aspects, the method of making the flow cell article can further include controlling the density of the nucleic acid primer molecules 42 by determining and selecting, in advance, by for example, stoichiometry, the ratio of polymer to nucleic acid primer molecules 42.

In additional aspects of the present disclosure, the disclosure provides a method for sequencing a nucleic acid, the method including: providing the flow cell article 10 having: the substrate 14 having one or more layers; the fluidic channel 18 disposed in the substrate 14 wherein the fluidic channel 18 includes at least one reactive surface 22 including the coupling agent 26 having the first functional group 30 covalently attached to the substrate 14 of the fluidic channel 18 and the second functional group 34 covalently attached to a polymer of formula (I) having an imide functional linker: 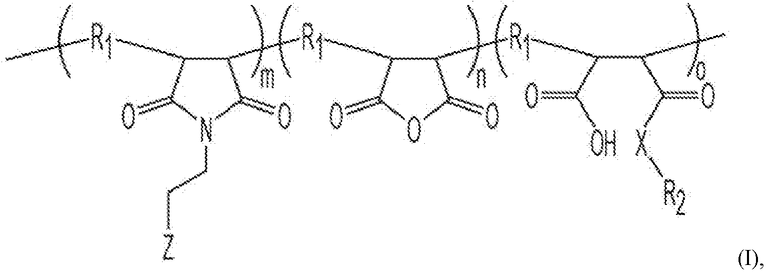 where: R₁ is a residue of an unsaturated monomer that has been copolymerized with maleic anhydride; R₂ is H, an alkyl group, an oligo(ethylene glycol), and/or a dialkyl amine; m, n, and o are each from 1 to 10,000; X is a divalent NH, O, and/or S; and Z is the first functional group 30. The method for sequencing a nucleic acid further includes contacting the nucleic acid primer molecule 42 with the tethered polymer 38 to covalently attached the nucleic acid primer molecule 42 to the tethered polymer 38.

In some aspects, once the flow cell article 10 is produced, a method used to sequence nucleic acid segments can be implemented. The method further includes contacting the nucleic acid primer molecule 42 with the polymer 38 of formula (I) to covalently attach the nucleic acid primer molecule 42 to the polymer 38 of formula (I); capturing DNA fragments using the nucleic acid primer molecule 42, wherein each DNA fragment contains a complementary sequence to the nucleic acid primer molecule 42; and adding nucleotides to the end of the nucleic acid primer molecule 42 to synthesize a complementary DNA sequence for each DNA fragment captured where the complementary DNA sequence is covalently coupled to the polymer 38 of formula (I) through the nucleic acid primer molecule 42. In some aspects, the method used to sequence nucleic acid segments may further include denaturing the complementary DNA sequence from the DNA fragment; collecting the DNA fragment from the flow cell article; synthesizing target DNA fragments using the complementary DNA sequence covalently coupled to the polymer of formula (I) through the nucleic acid primer molecule 42; and collecting the target DNA fragments from the flow cell article 10.

In some aspects, the step of capturing DNA fragments, each containing a complementary sequence to the nucleic acid primer molecule 42, from a sample and covalently adding nucleotides from the end of the primer molecule 42 can be used to synthesize a complementary DNA sequence for each captured fragment. These complementary DNA fragments can be individually or separately covalently coupled to the tethered polymer as desired by the application. The double stranded DNA, the complementary DNA sequence and DNA fragment captured, may then be denatured and the corresponding strands can be collected and removed. In some aspects, after the DNA fragments are removed, each complementary DNA or DNA fragment can be locally amplified to form a monoclonal cluster using polymerase chain reactions. Depending on resolution of the optical system used for fluorescence imaging, the resulted clusters can have a size of 50 nm to 1000 nm in diameter. This synthetic method provides a technique that can be useful for sequencing-by-synthesis based next generation sequencing (NGS) techniques, where polyclonal clusters are generally formed before sequencing. The cluster generation can be achieved using, for example, bridge amplification, exclusion amplification, or template walking approach, after the nucleic acid primer molecules 42 and DNA molecules are coupled to the fluidic channel 18 of the substrate 14.

It is understood that the descriptions outlining and teaching the flow cell article 10 previously discussed, which can be used in any combination, apply equally well to the methods used to sequence nucleic acid segments.

The present disclosure enables rapid covalent coupling of amine-terminated nucleic acid primer molecules 42 (e.g., 5'-amine-dA30, or 5'-amine-dT30) to the substrate 14. The coupling can be completed, for example, within one hr.

The present disclosure provides a more stable attachment of nucleic acid primer molecules 42 onto the substrate, compared to the coupling using a bifunctional linker or other means. This is in part due to the multivalent, strong anchorage of the polymer layer to the amine presenting surface (e.g., APTES), and in part due to the imide bond formation. This stable bonding attachment is significant, given that DNA sequencing often needs to run many reaction cycles, and involves some harsh treatments such as NaOH rinsing to denature any duplex DNA before sequencing read. The use of imide linkages formed by reacting the succinic anhydride functional group with primary amine built into the coupling agent 26 and/or nucleic acid primer molecules 42 provide a chemical resistant and stable covalent chemical linkage which is resistant to degradative loss or surface separation arising from these harsh chemical treatments used in sequencing chemistries.

The present disclosure also provides a method of precise control of (and ultimately more efficient) hybridization between the nucleic acid primer molecule 42 and a target DNA fragment containing an adaptor sequence complementary to the probe. This phenomenon is mostly due to the flexibility of the polymer chains even after coating. In contrast, for the bifunctional linker based DNA attachment, these DNA molecules are very close to the surface, thus preventing efficient hybridization.

The disclosure also provides precise control of the density of the nucleic acid primer molecules 42 attached to the surface, which permits user determined adjustability of the DNA hybridization efficiency, and subsequent clustering efficiency. This control can be achieved at either of two different chemical reactions. First, the amine reactive polymer is partially modified and derivatized with the surface modifying molecule 50 (e.g., propylamine or the like). This functionalization can help control the solubility of the polymer 38 in solution for coating and the reactive sites once attached to the coupling agent 26 of the substate 14. Second, the amine-terminated nucleic acid primer molecules 42 may be incubated with the amine reactive polymer 38 modified surface in the presence of the surface modifying molecule 50, for example, ethanolamine or like agents, at a desired concentration. This step controls the covalent coupling reaction and, in turn, the density of the nucleic acid primer molecules 42 attached. The combination of these two different reactions permit excellent density control of the nucleic acid primer molecules 42 or DNA/RNA primer molecules attached to the fluidic channel 18 of the substrate 14, and ultimately the density of clusters formed, and the efficiency of the sequencing.

The present disclosure may also be applicable to NGS using nano-patterned flow cells. Nanopatterning can be achieved using state-of-the-art photolithography or nanoimprinting approaches. In some aspects, the flow cell article 10 may include an array of discrete spots of amine reactive polymer coating and covalently bound nucleic acid primer molecules 42. The nanopatterning can be achieved by, for example, state-of-the-art photolithography or nanoimprinting techniques. In alternative embodiments, low density of polymer nanoparticle coating can be used as a random patterned surface so that clusters formed can be confined with each polymer nanoparticle, and well separated from each other.

The present disclosure may also be applicable for different biomolecular analysis techniques using nucleic acid-based biosensors, where the density of the nucleic acid primer molecules 42 attached can be important in contributing to the success of bioassays.

It will be understood by one having ordinary skill in the art that construction of the described device and other components may not be limited to any specific material. Other exemplary embodiments of the device disclosed herein may be formed from a wide variety of materials, unless described otherwise herein.

For purposes of this disclosure, the term "coupled" (in all of its forms, couple, coupling, coupled, etc.) generally means the joining of two components (electrical or mechanical) directly or indirectly to one another. Such joining may be stationary in nature or movable in nature. Such joining may be achieved with the two components (electrical or mechanical) and any additional intermediate members being integrally formed as a single unitary body with one another or with the two components. Such joining may be permanent in nature or may be removable or releasable in nature unless otherwise stated.

It is also important to note that the construction and arrangement of the elements of the device as shown in the exemplary embodiments is illustrative only. Although only a few embodiments of the present innovations have been described in detail in this disclosure, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter recited. For example, elements shown as integrally formed may be constructed of multiple parts or elements shown as multiple parts may be integrally formed, the operation of the interfaces may be reversed or otherwise varied, the length or width of the structures and/or members or connector or other elements of the system may be varied, the nature or number of adjustment positions provided between the elements may be varied. It should be noted that the elements and/or assemblies of the system may be constructed from any of a wide variety of materials that provide sufficient strength or durability, in any of a wide variety of colors, textures, and combinations. Accordingly, all such modifications are intended to be included within the scope of the present innovations. Other substitutions, modifications, changes, and omissions maybe made in the design, operating conditions, and arrangement of the desired and other exemplary embodiments without departing from the present innovations.

It will be understood that any described processes or steps within described processes may be combined with other disclosed processes or steps to form structures within the scope of the present device. The exemplary structures and processes disclosed herein are for Page 21 of 30 illustrative purposes and are not to be construed as limiting.

It is also to be understood that variations and modifications can be made on the aforementioned structure without departing from the concepts of the present disclosure, and further it is to be understood that such concepts are intended to be covered by the following claims unless these claims by their language expressly state otherwise.

The above description is considered that of the illustrated embodiments only. Modifications of the device will occur to those skilled in the art and to those who make or use the device. Therefore, it is understood that the embodiments shown in the drawings and described above is merely for illustrative purposes and not intended to limit the scope of the device, which is defined by the following claims as interpreted according to the principles of patent law, including the Doctrine of Equivalents.

## Claims

1. A flow cell article comprising:
a substrate comprising one or more layers;
a fluidic channel disposed in or on the substrate and comprising at least one reactive surface comprising:
a coupling agent comprising a first functional group covalently attached to the substrate and a second functional group covalently attached using at least one imide bond to a polymer of formula (I): 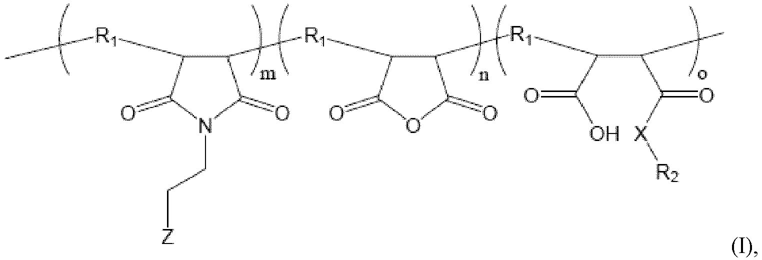
wherein:
R₁ is a residue of an unsaturated monomer that has been copolymerized with maleic anhydride;
R₂ is H, an alkyl group, an oligo(ethylene glycol), and/or a dialkyl amine;
m, n, and o are each from 1 to 10,000;
X is a divalent NH, O, and/or S;
and Z is the first functional group.

2. The flow cell article of claim 1, further comprising a nucleic acid primer molecule covalently attached to the polymer.

3. A flow cell system comprising:
a substrate comprising one or more layers;
a fluidic channel disposed in or on the substrate and comprising at least one reactive surface comprising:
a coupling agent comprising a first functional group covalently attached to the substrate of the fluidic channel and a second functional group positioned away from the substrate;
a polymer of formula (II) covalently attached to the second functional group of the coupling agent using at least one imide bond: 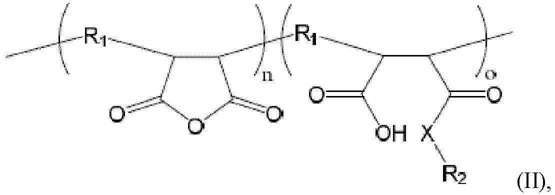
wherein:
R₁ is a residue of an unsaturated monomer that has been copolymerized with maleic anhydride;
R₂ is H, an alkyl group, an oligo(ethylene glycol), and/or a dialkyl amine;
n and o are each an integer from 1 to 10,000; and
X is a divalent NH, O, and/or S; and
a nucleic acid primer molecule covalently attached to the polymer.

4. The flow cell system of claim 2 or claim 3, wherein the nucleic acid primer molecule comprises an amine-terminated nucleic acid or a mixture of amine-terminated nucleic acids thereof.

5. The flow cell system of any of claims 2 to 4, wherein the nucleic acid primer molecule has a density of 1 to 500,000 probe molecules per square micrometer of surface area.

6. The flow cell article of any of claims 1 to 5, wherein the substrate comprises a glass, a glass ceramic, a silicon, a fused silica, a quartz, a thermoplastic, or a thermoset plastic.

7. The flow cell article of any of claims 1 to 6, wherein the coupling agent comprises an amino silane and/or an amino organophosphate.

8. The flow cell article of claim 7, wherein the amino silane is selected from the group consisting of 3-aminopropyltrimethoxylsilane, 3-aminopropyltriethoxysilane, 3-(2- aminoethyl)-aminopropyltrimethoxysilane, aminopropylmethyldialkoxysilanes, 4-aminobutyltriethoxysilane, 4-amino-3,3-dimethylbutyltrimethoxysilane, and N-(6-aminohexyl)aminomethyltriethoxysilane.

9. The flow cell article of claim 7, wherein the amino organophosphate is selected from the group consisting of 3-aminopropyl dihydrogen phosphate, 4-aminophenyl phosphate, 2-aminoethyl dihydrogen phosphate, and 2-(3-aminopropyl)aminoethyl phosphorothioate.

10. The flow cell system of any of claims 1 to 9, wherein a relative ratio of m to n (m:n) is from about 0.5 to about 10.

11. A method of making the flow cell article of any of claims 3 to 10, the method comprising:
contacting the fluidic channel disposed in or on the substrate with the coupling agent to covalently attach the first functional group to the fluidic channel;
contacting the polymer of formula (II) with the coupling agent to covalently attach the polymer to the second functional group to form an imide linkage on a tethered polymer; 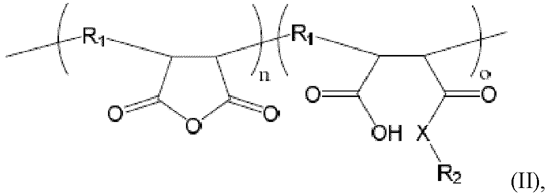
wherein:
R₁ is a residue of an unsaturated monomer that has been copolymerized with maleic anhydride;
R₂ is H, an alkyl group, an oligo(ethylene glycol), and/or a dialkyl amine;
n and o are each an integer from 1 to 10,000; and
X is a divalent NH, O, and/or S; and
contacting the nucleic acid primer molecule with the tethered polymer to covalently attach the nucleic acid primer molecule to the tethered polymer.

12. A method for sequencing nucleic acids using the flow cell of any of claims 1 to 2 and 4 to 10, the method comprising
contacting a nucleic acid primer molecule with the polymer of formula (I) to covalently attach the nucleic acid primer molecule to the polymer of formula (I);
capturing DNA fragments using the nucleic acid primer molecule, wherein each DNA fragment comprises a complementary sequence to the nucleic acid primer molecule; and
adding nucleotides to the end of the nucleic acid primer molecule to synthesize a complementary DNA sequence for each DNA fragment captured, wherein the complementary DNA sequence is covalently coupled to the polymer of formula (I) through the nucleic acid primer molecule.

13. The method of claim 12, wherein the nucleic acid probe comprises an amine-terminated nucleic acid or a mixture of amine-terminated nucleic acids thereof.

14. The method of claim 12 or claim 13, wherein the nucleic acid probe molecule has a density of 1 to 500,000 probe molecules per square micrometer of surface area.

15. The method of claim 12 or claim 13, further comprising:
denaturing the complementary DNA sequence from the DNA fragment; and
collecting the complementary DNA sequence and/or the DNA fragment from the flow cell article.

16. The method of claim 12 or claim 13, further comprising:
denaturing the complementary DNA sequence from the DNA fragment;
collecting the DNA fragment from the flow cell article;
synthesizing target DNA fragments using the complementary DNA sequence covalently coupled to the polymer of formula (I) through the nucleic acid primer molecule; and
collecting the target DNA fragments from the flow cell article.

## Patentansprüche

1. Durchflusszellenartikel, umfassend:
ein Substrat, das eine oder mehrere Schichten umfasst;
einen Fluidkanal, der in oder auf dem Substrat angeordnet ist und mindestens eine reaktive Oberfläche umfasst, umfassend:
ein Verbindungsmittel, das eine erste funktionelle Gruppe, die kovalent an das Substrat gebunden ist, und eine zweite funktionelle Gruppe umfasst, die unter Verwendung von mindestens einer Imidbindung kovalent an ein Polymer der Formel (I) gebunden ist: 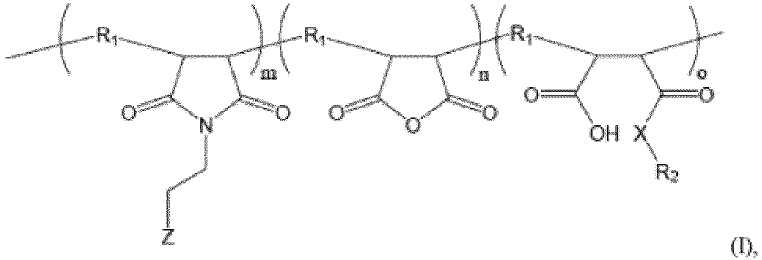
wobei:
R₁ ein Rest eines ungesättigten Monomers ist, das mit Maleinsäureanhydrid copolymerisiert wurde;
R₂ H, eine Alkylgruppe, ein Oligo(ethylenglycol) und/oder ein Dialkylamin ist;
m, n und o jeweils 1 bis 10.000 sind;
X ein zweiwertiges NH, O und/oder S ist;
und Z die erste funktionelle Gruppe ist.

2. Durchflusszellenartikel nach Anspruch 1, ferner umfassend ein Nukleinsäure-Primermolekül, das kovalent an das Polymer gebunden ist.

3. Durchflusszellensystem, umfassend:
ein Substrat, das eine oder mehrere Schichten umfasst;
einen Fluidkanal, der in oder auf dem Substrat angeordnet ist und mindestens eine reaktive Oberfläche umfasst, umfassend:
ein Verbindungsmittel, das eine erste funktionelle Gruppe, die kovalent an das Substrat des Fluidkanals gebunden ist, und eine zweite funktionelle Gruppe umfasst, die von dem Substrat entfernt angeordnet ist;
ein Polymer der Formel (II), das unter Verwendung mindestens einer Imidbindung kovalent an die zweite funktionelle Gruppe des Verbindungsmittels gebunden ist: 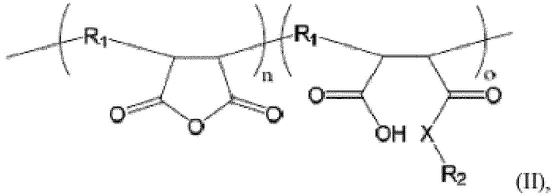
wobei:
R₁ ein Rest eines ungesättigten Monomers ist, das mit Maleinsäureanhydrid copolymerisiert wurde;
R₂ H, eine Alkylgruppe, ein Oligo(ethylenglycol) und/oder ein Dialkylamin ist;
n und o jeweils eine ganze Zahl von 1 bis 10.000 sind; und
X ein zweiwertiges NH, O und/oder S ist; und
ein Nukleinsäure-Primermolekül, das kovalent an das Polymer gebunden ist.

4. Durchflusszellensystem nach Anspruch 2 oder Anspruch 3, wobei das Nukleinsäure-Primermolekül eine Amin-terminierte Nukleinsäure oder ein Gemisch von Amin-terminierten Nukleinsäuren davon umfasst.

5. Durchflusszellensystem nach einem der Ansprüche 2 bis 4, wobei das Nukleinsäure-Primermolekül eine Dichte von 1 bis 500.000 Sondenmolekülen pro Quadratmikrometer Oberfläche aufweist.

6. Durchflusszellenartikel nach einem der Ansprüche 1 bis 5, wobei das Substrat ein Glas, eine Glaskeramik, ein Silizium, ein Quarzglas, einen Quarz, einen thermoplastischen oder einen duroplastischen Kunststoff umfasst.

7. Durchflusszellenartikel nach einem der Ansprüche 1 bis 6, wobei das Verbindungsmittel ein Aminosilan und/oder ein Aminoorganophosphat umfasst.

8. Durchflusszellenartikel nach Anspruch 7, wobei das Aminosilan aus der Gruppe ausgewählt ist, die aus 3-Aminopropyltrimethoxysilan, 3-Aminopropyltriethoxysilan, 3-(2-Aminoethyl)-aminopropyltrimethoxysilan, Aminopropylmethyldialkoxysilanen, 4-Aminobutyltriethoxysilan, 4-Amino-3,3-Dimethylbutyltrimethoxysilan und N-(6-Aminohexyl)aminomethyltriethoxysilan besteht.

9. Durchflusszellenartikel nach Anspruch 7, wobei das Aminoorganophosphat aus der Gruppe ausgewählt ist, die aus 3-Aminopropyldihydrogenphosphat, 4-Aminophenylphosphat, 2-Aminoethyldihydrogenphosphat und 2-(3-Aminopropyl)aminoethylphosphorothioat besteht.

10. Durchflusszellensystem nach einem der Ansprüche 1 bis 9, wobei ein relatives Verhältnis von m zu n (m:n) etwa 0,5 bis etwa 10 beträgt.

11. Verfahren zum Herstellen des Durchflusszellenartikels nach einem der Ansprüche 3 bis 10, wobei das Verfahren umfasst:
Inkontaktbringen des Fluidkanals, der in oder auf dem Substrat angeordnet ist, mit dem Verbindungsmittel, um die erste funktionelle Gruppe kovalent an den Fluidkanal zu binden;
Inkontaktbringen des Polymers der Formel (II) mit dem Verbindungsmittel, um das Polymer kovalent an die zweite funktionelle Gruppe zu binden, um eine Imidbindung an einem verknüpften Polymer zu bilden; 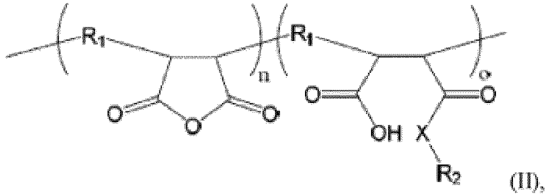
wobei:
R₁ ein Rest eines ungesättigten Monomers ist, das mit Maleinsäureanhydrid copolymerisiert wurde;
R₂ H, eine Alkylgruppe, ein Oligo(ethylenglycol) und/oder ein Dialkylamin ist;
n und o jeweils eine ganze Zahl von 1 bis 10.000 sind; und
X ein zweiwertiges NH, O und/oder S ist; und
Inkontaktbringen des Nukleinsäure-Primermoleküls mit dem verknüpften Polymer, um das Nukleinsäure-Primermolekül kovalent an das verknüpfte Polymer zu binden.

12. Verfahren zum Sequenzieren von Nukleinsäuren unter Verwendung der Durchflusszelle nach einem der Ansprüche 1 bis 2 und 4 bis 10, wobei das Verfahren umfasst Inkontaktbringen eines Nukleinsäure-Primermoleküls mit dem Polymer der Formel (I), um das Nukleinsäure-Primermolekül kovalent an das Polymer der Formel (I) zu binden;
Einfangen von DNA-Fragmenten unter Verwendung des Nukleinsäure-Primermoleküls, wobei jedes DNA-Fragment eine komplementäre Sequenz zu dem Nukleinsäure-Primermolekül umfasst; und
Hinzufügen von Nukleotiden an das Ende des Nukleinsäure-Primermoleküls, um eine komplementäre DNA-Sequenz für jedes eingefangene DNA-Fragment zu synthetisieren, wobei die komplementäre DNA-Sequenz durch das Nukleinsäure-Primermolekül kovalent an das Polymer der Formel (I) gekoppelt ist.

13. Verfahren nach Anspruch 12, wobei die Nukleinsäuresonde eine Amin-terminierte Nukleinsäure oder ein Gemisch von Amin-terminierten Nukleinsäuren davon umfasst.

14. Verfahren nach Anspruch 12 oder Anspruch 13, wobei das Nukleinsäure-Sondenmolekül eine Dichte von 1 bis 500.000 Sondenmolekülen pro Quadratmikrometer Oberfläche aufweist.

15. Verfahren nach Anspruch 12 oder Anspruch 13, ferner umfassend:
Denaturieren der komplementären DNA-Sequenz aus dem DNA-Fragment; und
Sammeln der komplementären DNA-Sequenz und/oder des DNA-Fragments aus dem Durchflusszellenartikel.

16. Verfahren nach Anspruch 12 oder Anspruch 13, ferner umfassend:
Denaturieren der komplementären DNA-Sequenz aus dem DNA-Fragment;
Sammeln des DNA-Fragments aus dem Durchflusszellenartikel;
Synthetisieren von Ziel-DNA-Fragmenten unter Verwendung der komplementären DNA-Sequenz, die durch das Nukleinsäure-Primermolekül kovalent an das Polymer der Formel (I) gekoppelt ist; und
Sammeln der Ziel-DNA-Fragmente aus dem Durchflusszellenartikel.

## Revendications

1. Article à cellule d'écoulement comprenant :
un substrat comprenant une ou plusieurs couches ;
un canal fluidique disposé dans le substrat ou sur celui-ci et comprenant au moins une surface réactive comprenant :
un agent de couplage comprenant un premier groupe fonctionnel lié par covalence au substrat et un second groupe fonctionnel lié par covalence à l'aide d'au moins une liaison imide à un polymère de formule (I) : 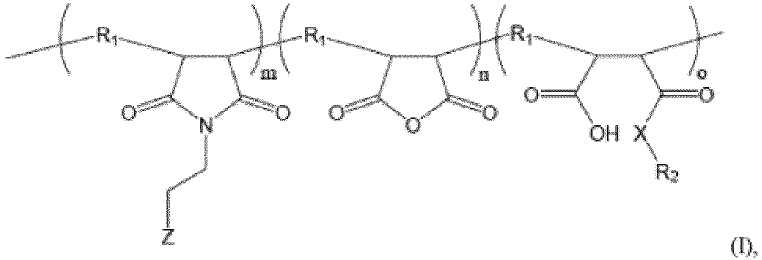
dans laquelle :
R₁ représente un résidu d'un monomère insaturé qui a été copolymérisé avec un anhydride maléique ;
R₂ représente un atome H, un groupe alkyle, un oligo(éthylène glycol) et/ou une dialkylamine ;
m, n et o valent chacun de 1 à 10 000 ;
X représente un groupe NH, un atome O et/ou un atome S divalents ;
et Z représente le premier groupe fonctionnel.

2. Article à cellule d'écoulement selon la revendication 1, comprenant en outre une molécule d'amorce d'acide nucléique liée par covalence au polymère.

3. Système à cellule d'écoulement comprenant :
un substrat comprenant une ou plusieurs couches ;
un canal fluidique disposé dans le substrat ou sur celui-ci et comprenant au moins une surface réactive comprenant :
un agent de couplage comprenant un premier groupe fonctionnel lié par covalence au substrat du canal fluidique et un second groupe fonctionnel positionné à distance du substrat ;
un polymère de formule (II) lié par covalence au second groupe fonctionnel de l'agent de couplage à l'aide d'au moins une liaison imide : 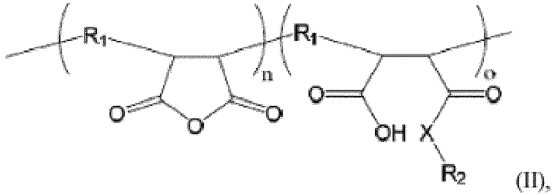
dans laquelle :
R₁ représente un résidu d'un monomère insaturé qui a été copolymérisé avec un anhydride maléique ;
R₂ représente un atome H, un groupe alkyle, un oligo(éthylène glycol), et/ou une dialkylamine ;
n et o représentent chacun un entier de 1 à 10 000 ; et
X représente un groupe NH, un atome O et/ou un atome S divalents ; et
une molécule d'amorce d'acide nucléique liée par covalence au polymère.

4. Système à cellule d'écoulement selon la revendication 2 ou la revendication 3, ladite molécule d'amorce d'acide nucléique comprenant un acide nucléique à terminaison amine ou un mélange d'acides nucléiques à terminaison amine de celle-ci.

5. Système à cellule d'écoulement selon l'une quelconque des revendications 2 à 4, ladite molécule d'amorce d'acide nucléique présentant une densité de 1 à 500 000 molécules sondes par micromètre carré de surface.

6. Article à cellule d'écoulement selon l'une quelconque des revendications 1 à 5, ledit substrat comprenant un verre, une vitrocéramique, un silicium, une silice fusionnée, un quartz, un thermoplastique ou un plastique thermodurci.

7. Article à cellule d'écoulement selon l'une quelconque des revendications 1 à 6, ledit agent de couplage comprenant un aminosilane et/ou un aminoorganophosphate.

8. Article à cellule d'écoulement selon la revendication 7, ledit aminosilane étant choisi dans le groupe constitué par le 3-aminopropyltriméthoxylsilane, le 3-aminopropyltriéthoxysilane, le 3-(2-aminoéthyl)-aminopropyltriméthoxysilane, les aminopropylméthyldialcoxysilanes, le 4-aminobutyltriéthoxysilane, le 4-amino-3,3-diméthylbutyltriméthoxysilane et le N-(6-aminohexyl)aminométhyltriéthoxysilane.

9. Article à cellule d'écoulement selon la revendication 7, ledit aminoorganophosphate étant choisi dans le groupe constitué par le dihydrogénophosphate de 3-aminopropyle, le phosphate de 4-aminophényle, le dihydrogénophosphate de 2-aminoéthyle et le phosphorothioate de 2-(3-aminopropyl)aminoéthyle.

10. Système à cellule d'écoulement selon l'une quelconque des revendications 1 à 9, le rapport relatif de m à n (m:n) valant d'environ 0,5 à environ 10.

11. Procédé de fabricationd'un article à cellule d'écoulement selon l'une quelconque des revendications 3 à 10, le procédé comprenant :
la mise en contact du canal fluidique disposé dans le substrat ou sur celui-ci avec l'agent de couplage pour lier par covalence le premier groupe fonctionnel au canal fluidique ;
la mise en contact du polymère de formule (II) avec l'agent de couplage pour lier par covalence le polymère au second groupe fonctionnel pour former une liaison imide sur un polymère captif ; 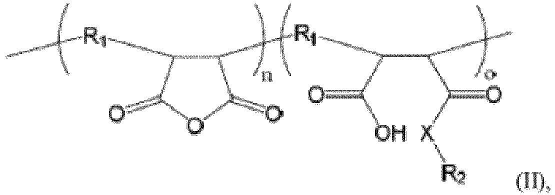
dans laquelle :
R₁ représente un résidu d'un monomère insaturé qui a été copolymérisé avec un anhydride maléique ;
R₂ représente un atome H, un groupe alkyle, un oligo(éthylène glycol) et/ou une dialkylamine ;
n et o représentent chacun un entier de 1 à 10 000 ; et
X représente un groupe NH, un atome O et/ou un atome S divalents ; et
la mise en contact de la molécule d'amorce d'acide nucléique avec le polymère captif pour lier par covalence la molécule d'amorce d'acide nucléique au polymère captif.

12. Procédé de séquençage d'acides nucléiques à l'aide d'une cellule d'écoulement selon l'une quelconque des revendications 1 à 2 et 4 à 10, le procédé comprenant
la mise en contact d'une molécule d'amorce d'acide nucléique avec le polymère de formule (I) pour lier par covalence la molécule d'amorce d'acide nucléique au polymère de formule (I) ;
la capture de fragments d'ADN à l'aide de la molécule d'amorce d'acide nucléique, chaque fragment d'ADN comprenant une séquence complémentaire à la molécule d'amorce d'acide nucléique ; et
l'ajout de nucléotides à l'extrémité de la molécule d'amorce d'acide nucléique pour synthétiser une séquence d'ADN complémentaire pour chaque fragment d'ADN capturé, ladite séquence d'ADN complémentaire étant couplée par covalence au polymère de formule (I) par l'intermédiaire de la molécule d'amorce d'acide nucléique.

13. Procédé selon la revendication 12, ladite sonde d'acide nucléique comprenant un acide nucléique à terminaison amine ou un mélange d'acides nucléiques à terminaison amine de celle-ci.

14. Procédé selon la revendication 12 ou la revendication 13, ladite molécule sonde d'acide nucléique présentant une densité de 1 à 500 000 molécules sondes par micromètre carré de surface.

15. Procédé selon la revendication 12 ou la revendication 13, comprenant en outre :
la dénaturation de la séquence d'ADN complémentaire provenant du fragment d'ADN ; et
la collecte de la séquence d'ADN complémentaire et/ou du fragment d'ADN à partir de l'article à cellule d'écoulement.

16. Procédé selon la revendication 12 ou la revendication 13, comprenant en outre :
la dénaturation de la séquence d'ADN complémentaire provenant du fragment d'ADN ;
la collecte du fragment d'ADN à partir de l'article à cellule d'écoulement ;
la synthèse des fragments d'ADN cibles à l'aide de la séquence d'ADN complémentaire couplée par covalence au polymère de formule (I) par l'intermédiaire de la molécule d'amorce d'acide nucléique ; et
la collecte des fragments d'ADN cibles à partir de l'article à cellule d'écoulement.
